Europäisches Patentamt

European Patent Office

Office européen des brevets

(19)

(11) Veröffentlichungsnummer : **0 275 817 B1**

(12) # EUROPÄISCHE PATENTSCHRIFT

(45) Veröffentlichungstag der Patentschrift :
05.06.91 Patentblatt 91/23

(51) Int. Cl.$^5$ : **A61N 5/08**

(21) Anmeldenummer : **87810043.7**

(22) Anmeldetag : **23.01.87**

(54) Elektrisches Lichtbad.

(43) Veröffentlichungstag der Anmeldung :
27.07.88 Patentblatt 88/30

(45) Bekanntmachung des Hinweises auf die
Patenterteilung :
05.06.91 Patentblatt 91/23

(84) Benannte Vertragsstaaten :
**AT BE CH DE FR GB IT LI LU NL SE**

(56) Entgegenhaltungen :
**EP-A- 0 023 311**
**WO-A-81/00677**
**CH-A- 441 526**
**DE-A- 3 103 731**

(56) Entgegenhaltungen :
**DE-A- 3 127 054**
**FR-A- 1 184 256**
**GB-A- 262 251**
**US-A- 3 118 065**

(73) Patentinhaber : **Wolff, Friedrich**
**Störklingasse 38**
**CH-4125 Riehen/Basel (CH)**

(72) Erfinder : **Wolff, Friedrich**
**Störklingasse 38**
**CH-4125 Riehen/Basel (CH)**

(74) Vertreter : **Eschmann, Heinz et al**
**A. Braun, Braun, Héritier, Eschmann AG**
**Patentanwälte Holbeinstrasse 36-38**
**CH-4051 Basel (CH)**

**Beschreibung**

Die Erfindung bezieht sich auf ein elektrisches-Lichtbad gemäss dem Oberbegriff des Anspruchs 1.

Ein bekanntes derartiges elektrisches Lichtbad (WO/81/00677) weist ein Bestrahlungsgerät auf, bei dem innerhalb eines als Paraboloid oder Ellipsoid ausgebildeten Reflektors eine Glühfadenlampe als Infrarotlichtquelle angeordnet ist.

Damit nur Infrarotstrahlen einer vorgebbaren Wellenlänge austreten, sind Infrarotfilter angeordnet. Damit lässt sich eine Strahlung erzeugen, mit der auch während längerer Zeit ein Schwitzen erzielt werden kann, ohne dass eine unangenehme Hitzeempfindung auftritt.

Die Infrarotlichtquelle des bekannten elektrischen Lichtbades ist fokussierbar ; damit kann eine mit Strahlung zu beaufschlagende Fläche eines zu behandelnden menschlichen Körpers hinsichtlich ihrer Grösse verändert werden.

Die Wärmestrahlung einer Sauna hingegen ist langwellig, dringt wenig tief in die Haut, und führt zu einer Erwärmung des Körperkernes. Infolgedessen kommt es zu einer Erwärmung des Blutes. Dies wiederum hat zur Folge, dass das Blut weniger Sauerstoff zu binden vermag. Bei Hypotonikern oder nach Alkoholgenuss, nach welchem die Sauerstoffbindungsfähigkeit des Blutes schon ohnehin herabgesetzt ist, kann es leicht zu Schwindelgefühlen, zu Unwohlsein und sogar zu Bewusstlosigkeit kommen. Aber selbst bei guter körperlicher Verfassung kann die Sauna nur dosiert angewendet werden. Spätestens nach einer Schwitzperiode von maximal 15 bis 20 Minuten sollte jeweils eine Pause gemacht und die Wärmeapplikation gegebenenfalls anschliessend wiederholt werden.

Weitere Nachteile der Sauna sind der grosse Platzbedarf, die aufwendigen Installationen und nicht zuletzt der grosse Energiebedarf.

Demgegenüber geht der Einsatz eines elektrischen Lichtbades von der Überlegung aus, dass zum Schwitzen eigentlich in erster Linie die Haut erwärmt werden müsste, um auf diese Weise gezielt die Schweissdrüsen zur Tätigkeit anregen zu können, ohne dass der ganze Körper aufgeheizt würde.

Es sind weiterhin elektrische Lichtbäder bekannt, bei welchen ein grösserer Kasten mittels Glühlampen oder stromdurchflossener Drähte erwärmt wird. Der Kranke liegt oder sitzt in der Weise, dass der Kopf gekühlt werden kann. Diese Art Lichtbäder wird in der Regel subjektiv als äusserst unangenehm empfunden und bisher nur im Krankheitsfalle – insbesondere bei Rheumatismus – eingesetzt.

Des weiteren sind Infrarotstrahler, beispielsweise Quarz-Infrarot-Strahler, bekannt, welche lokal auf einzelne Körperteile, vorzugsweise auf Gelenke, gerichtet werden. Elektrische Lichtbäder dieser Art, bei denen bloss begrenzte Hautbereiche bestrahlt werden, können subjektiv durchaus als angenehm empfunden werden. Ein Schwitzen lässt sich jedoch kaum erreichen. Eine Erhöhung der Strahlungsintensität führt rasch zu einer unangenehmen Hitzeempfindung, bevor es zum erwünschten Schwitzen kommt.

Der Erfindung liegt die Aufgabe zugrunde mit einfachen Mitteln ein gattungsgemässes elektrisches Lichtbad so weiterzubilden, dass die auf einen seitlichen Teil eines zu behandelnden Körpers auftreffende Strahlung intensiviert wird, ohne dass dabei eine unangenehme Hitzeempfindung verursacht wird.

Diese Aufgabe wird bei einem gattungsgemässen Lichtbad durch die kennzeichnenden Merkmale des Anspruchs 1 gelöst.

Durch den erfindungsgemäss angeordneten Spalt wird es ermöglicht, dass ein Teil der von der Infrarotlichtquelle ausgehenden Strahlung auf die Zusatzreflektoren gelangt, um von dort seitlich auf den Körper gerichtet zu werden.

Vorteilhafte weitere Ausgestaltungen betreffend die Anordnung der beiden Zusatzreflektoren und der Infrarotlichtquelle sind in den Ansprüchen 2 bis 4 angegeben.

Bei einer bevorzugten Ausführungsform sind gemäss dem Anspruch 5 wenigstens zwei seitlich von der Infrarotquelle angeordnete, reflektierende Wände vorgesehen. Damit kann die seitlich auf einen Körper auftreffende Strahlung noch intensiviert werden.

Weitere vorteilhafte Ausgestaltungen ergeben sich aus den Ansprüchen 6 und 7.

Nach längeren Untersuchungen zeigte es sich, dass bei einer vorgegebenen, noch als angenehm empfundenen, auf den Körper einwirkenden Strahlungsintensität, ein Mindestanteil der Körperoberfläche, vorzugsweise etwa zwei Drittel bestrahlt werden müssen, damit das gewünschte Schwitzen auftritt. Diese Art zu Schwitzen ist sehr angenehm, da der Körperkern nicht aufgeheizt wird. Ja es ist sogar möglich, im Anschluss an eine Saunabad ohne weiteres ein längeres Lichtbad der erfindungsgemässen Art zu nehmen.

Wird hingegen ein zu grosser Anteil der Körperoberfläche bestrahlt, so wird auch eine minimale Strahlungsintensität, welche eben noch zum Schwitzen führt, subjektiv als unangenehm empfunden. Es ist also wesentlich, dass ein nicht zu kleiner und nicht zu grosser Hautbereich bestrahlt wird.

Zusammenfassend lassen sich also beim erfindungsgemässen Lichtbad insbesondere folgende Vorteile nennen :

1. Die oben genannten Vorteile des Schwitzbades : Aehnlich wie an der Sonne gerät der Körper innerhalb weniger Minuten kräftig ins Schwitzen. Da die Erwärmung nicht durch heisse Luft bzw. die sehr langewellige Abstrahlung von Hohlwänden, sondern durch die tief in die Haut eindringenden IR-Strahlen erzeugt wird, entsteht keine Kreislaufbelastung. Das Schwitzbad muss deshalb nicht 2-3 mal nach Abkühlung wiederholt werden. Es wirkt entschlackend und entwässernd. Kombiniert mit einer Diätkur, führt es nach wenigen Tagen zu signifikanter Gewichtsabnahme.

2. Hinzu kommen die Vorteile einer IR-Behandlung : Infrarotstrahlen werden zur Behandlung vielfältiger Erkrankungen eingesetzt, so zB. bei Rheuma, Ischias, Gicht, Muskelschmerzen, Neuralgie, Hautverletzungen, Blutergüssen, Verstauchungen, Prellungen und vieles mehr. Die jetzt mögliche Ganzkörperbestrahlung erweitert die Anwendungsmöglichkeiten : Durchblutungs- und Stoffwechselsteigerungen des gesamten Hautorgans, die Haut reinigt und strafft sich.

3. Wenn auch der sichtbare, insbesondere der hellorange Lichtbereich angewendet wird, wird die Bildung von Melatonin gehemmt, welche in Zeiten mit wenig äusserem Licht verstärkt auftritt und zur sogenannten Winterdepression führt.

4. Eine einfache Montage und sofortige Betriebsbereitschaft erlauben eine problemlose Anwendung in vielen Fällen, in welchen eine Sauna infolge des damit verbundenen Aufwandes nicht ohne weiteres in Frage kommt, beispielsweise beim Physiotherapeuten, beim Friseur und in der Etagenwohnung. Die Anwendung ist physiologisch angenehm. Die Funktionsaktivierung der elastischen Fasern der Haut führt zu deren Straffung, so dass auch die Anwendung im Schönheitssalon in Frage kommt.

Nachstehend wird die Erfindung anhand einer Zeichnung beispielsweise näher erläutert. Es zeigen:

Fig. 1 eine schematische Darstellung einer bevorzugten Ausführungsform einer erfindungsgemässen Infrarotlichtquelle im Querschnitt.

Fig. 2 eine entsprechende Darstellung im Längsschnitt

Fig. 3 eine diesbezügliche Kabine mit reflektierenden Wänden

Fig. 4 den Intensitätsverlauf der Infrarotstrahlung in Abhängigkeit der Wellenlänge

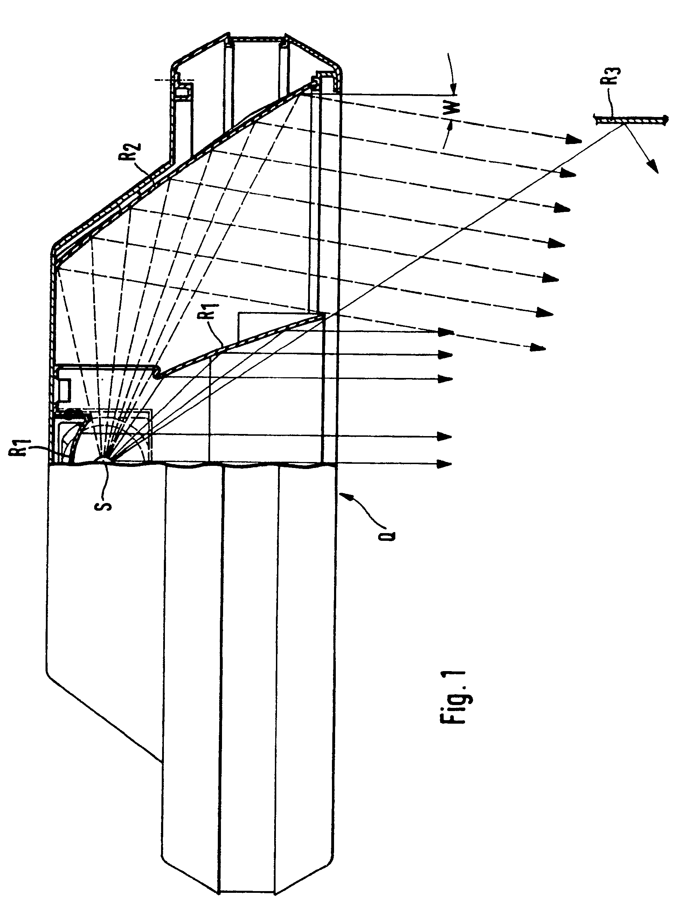

Figur 1 zeigt eine bevorzugte Ausführungsform der Erfindung. Eine Infrarotlichtquelle Q ist hälftig im Querschnitt (rechts) und hälftig in der Aufsicht auf die Breitseite gezeigt. Ein Infrarotheizstrahlerelement S wirft seine Strahlung auf einen ersten Reflektor R1. Dieser ist parabolisch geformt, wobei sich das Strahlerelement S in dessen Brennpunkt befindet. Infolgedessen wird die Strahlung parallel nach unten reflektiert. Die Breite dieses Reflektors ist wenigstens so breit, dass eine darunter befindliche Person von der Strahlung vollständig erfasst wird. Zur Klärung der Terminologie sei an dieser Stelle vermerkt, dass in dieser Schrift unter "Strahlerelement S" das handelsübliche Bauteil zur Erzeugung von IR-Strahlung und unter "Infrarotlichtquelle Q" das gesamte Strahlergerät, inklusive Strahlerelement S, Gehäuse und weiteren Bauteilen, wie Reflektoren, Lamellen etc. verstanden wird.

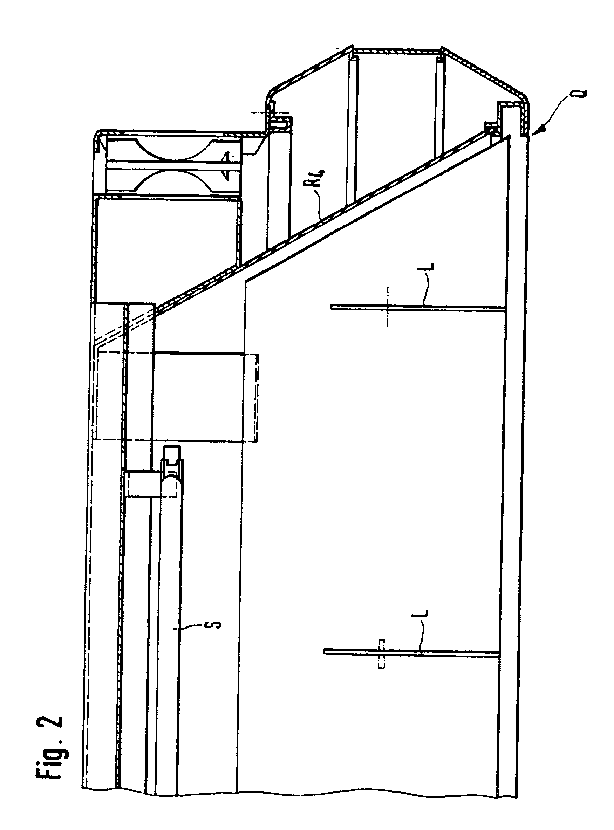

Im Reflektor R1 befindet sich seitlich etwa in Höhe des Strahlerelementes S ein Spalt, durch den ein Teil der Strahlung gelangen kann. Diese wird von je einem Zusatzreflektor R2 aufgefangen, welcher so geformt ist, dass die Strahlung in einem vorgegebenen, nach innen gerichteten Winkel W nach unten fällt. Infolge dieser Anwinkelung gelangt die Strahlung nicht nur frontal, sondern auch etwas seitlich auf den Körper eines in Figur 1 und 2 nicht dargestellten Anwenders. Auf diese Weise wird die auf den seitlichen Teil des Körpers auftreffende Strahlung intensiviert. Der äussere Abstand zwischen den beiden Zusatzreflektoren R2 ist einerseits möglichst breit zu wählen, damit ein möglichst grosser Strahlungswinkel W gebildet wird und die seitliche Strahlung möglichst steil auf den Körper auftrifft. Andererseits sollte dieser Abstand im Interesse eines möglichst kompakten Aufbaus der Infrarotlichtquelle Q nicht zu gross sein, so dass in der Praxis ein Abstand von 60 cm oder vorzugsweise 75 cm gewählt werden dürfte.

Mittels eines seitlich der Strahlungsquelle Q angeordneten, wenigstens annähernd vertikal ausgerichteten weiteren Reflektors R3 kann dieser Effekt noch intensiviert werden. Wie weiter unten noch ausgeführt werden wird, kann damit die seitlich auf den Körper auftreffende Strahlung noch intensiviert werden.

Figur 2 zeigt einen Teil des Längsschnittes der in Figur 1 gezeigten Infrarotquelle Q. Man erkennt wiederum ein Strahlerelement S. Ein Reflektor R4 bildet den Abschluss der Längsseite. Ferner sind zwei Lamellen L dargestellt, welche als Blendschutz wirken.

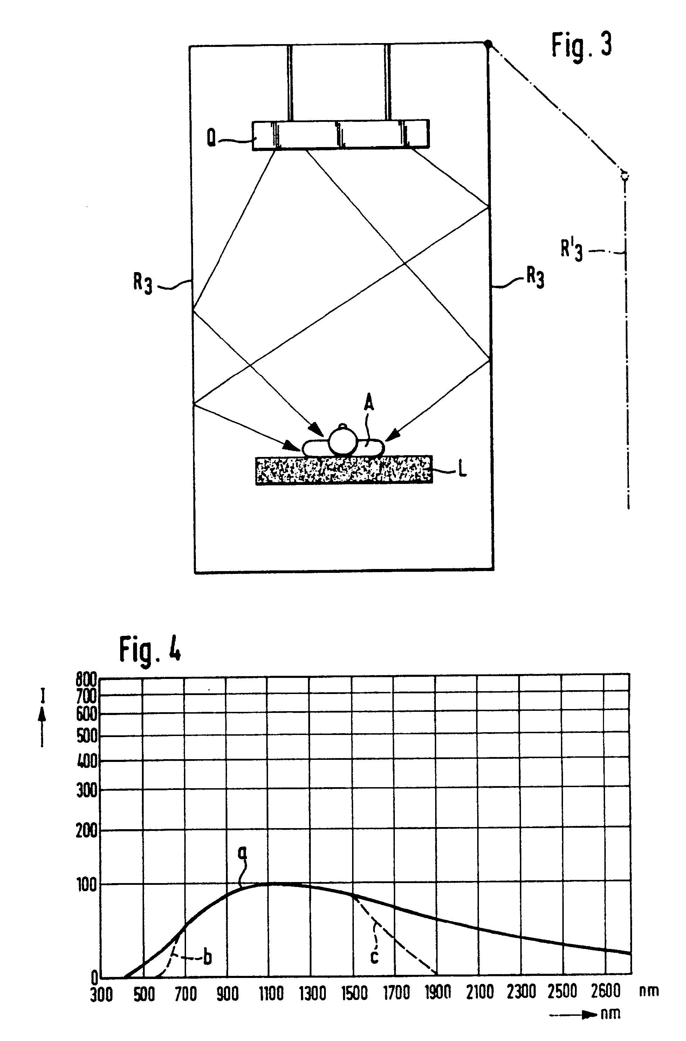

In Figur 3 ist ein komplettes Bestrahlungssystem mit Infrarotlichtquelle Q mit darunter befindlicher Liege L und mit reflektierenden Wänden R3 skizziert. Es ist insbesondere dargestellt, wie die aus der Infrarotlichtquelle Q austretende Strahlung auf den Anwender A nach Reflexion an den Seitenwänden R3 auch seitlich einstrahlt. Die reflektierenden Wände R3 sind in erster Linie seitlich der Infrarotlichtquelle Q und der Liege L angeordnet. Sie bilden beispielsweise Teil einer Bestrahlungskabine. Mit gestrichelter Linie R'3 ist angedeutet, dass wenigstens eine der Wände hochklappbar ausgebildet sein kann, um ein bequemes Betreten der Kabine zu ermöglichen. Eine Zugangsmöglichkeit kann aber auch mittels einer konventionellen Türe, mit Falt- Schiebe- oder sonstigen Vorrichtungen, welche nach innen mit einer reflektierenden Oberfläche versehen sind, verschafft werden.

In Figur 4 ist mit der Kurve a der Intensitätsverlauf eines handelsüblichen Infrarot-Heizstrahlerelementes in Abhängigkeit der Wellenlänge dargestellt. Kurve b zeigt den Verlauf mit einem das sichtbare Licht teilweise absorbierenden Filter, welches jedoch helloranges Licht noch durchlässt. Schliesslich zeigt die Kurve c den Verlauf mit einem Infrarot B und C absorbierenden Filter.

Wie einleitend bereits erwähnt wurde, ist es wichtig, dass einerseits ein Mindestbereich, andererseits aber doch nicht ein zu grosser Bereich der Körperoberfläche bestrahlt wird, damit ein subjektiv als angenehm empfundenes Schwitzen möglich ist. Demgegenüber ist es sekundär, wie die Infrarotquelle angeordnet wird, und ob der Anwender sitzt, steht oder liegt. In jedem Fall ist jedoch eine möglichst entspannende Lage anzustreben. Zu bevorzugen ist hierbei eine bequeme Liege, beispielsweise ein Wasserbett. Ein Badetuch fängt den Schweiss auf und dient der Hygiene.

Für die Anwendung mit einer Liege L wird die Infrarotquelle Q bevorzugt horizontal ausgerichtet, wie dies in Figur 3 gezeigt ist. Die Infrarotstrahlungsquelle Q ist beispielsweise 200 cm lang und mindestens 20 cm, vorteilhafterweise jedoch 50 bis 80 cm breit. Damit kann mittels des in Figur 1 gezeigten parabolischen Reflektors R1 eine senkrechte Bestrahlung der der Strahlungsquelle exponierten Körperseite erreicht werden.

Die durch den im Reflektor R1 vorgesehenen Spalt auf den weniger steil gerichteten Reflektor R2 fallende Strahlung sorgt für eine möglichst gleichmässige Bestrahlung aller 3 Körperseiten. Eine Intensivierung der naturgemäss etwas schwächer ausfallenden seitlichen Bestrahlung kann zusätzlich mit seitlich von Liege und Strahlungsquelle angeordneten reflektierenden Wänden R3 erreicht werden, wie dies in Figur 3 gezeigt ist. Beispielsweise können spezielle Reflektorwände aufgestellt werden, oder es werden die Innenseiten einer Schwitzbadkabine reflektierend ausgebildet.

Die Infrarotstrahlungsquelle Q wird vorteilhafterweise mit einzelnen stabförmigen Infrarotheizstrahlelementen S aufgebaut. Hierzu eignen sich beispielsweise Elemente des Typs 1-10-1000W der Firma Wolff. Bei einer Länge der Infrarotquelle von etwa 200 cm sind beim Ausführungsbeispiel drei Strahlerelemente dieses Typs erforderlich. Weil es wichtig ist, den Bereich zwischen Schwitzen und Einsetzen eines unangenehmen Hitzegefühls optimal zu erreichen, ist es vorteilhaft ein Steuergerät vorzusehen, mittels welchem der Anwender selbst die Bestrahlungsintensität individuell einstellen kann. Der Aufbau eines solchen Regelgerätes ist jedem Fachmann bestens bekannt und muss an dieser Stelle nicht behandelt werden. In einer bevorzugten Ausführungsform sind die Strahlerelemente einzeln regulierbar, so dass z.B. im Ausführungsbeispiel auf diese Weise der Kopf-Thorax-Bereich, der Rumpf und die Beine mit unterschiedlicher Intensität bestrahlt werden können.

Die Bestrahlungsstärke dürfte in einem Bereich zwischen 50 und 150 mW/cm², vorzugsweise jedoch zwischen 60 und 100 mW/cm² liegen.

Der Leistungsbedarf für eine solche Anlage liegt bei etwa 3000 Watt, so dass die Anlage an einer normalen Steckdose angeschlossen werden kann.

Um ein möglichst intensives Schwitzen ohne unangenehme Wärmeempfindung zu erreichen, wird die Infrarot B- und C-Strahlung möglichst unterdrückt. Hierfür existieren handelsübliche Filter. Gegebenenfalls kann auch eine für die Infrarotlichtquelle gegebenenfalls vorgesehene Glashülle entsprechend beschichtet werden.

Weil die Strahlungsquelle auch im sichtbaren Lichtbereich Strahlung abgibt, kann der Anwender geblendet werden. Um dem zu begegnen, können unterhalb der Strahlerelemente S Blendschutzlammellen L vorgesehen werden, wie dies in Figur 2 ausschnittsweise gezeigt ist.

Als andere oder zusätzliche Massnahme kann sichtbares Licht mit einem Filter reduziert werden, welches ebenfalls einer gegebenenfalls für die Strahlungsquelle vorgesehenen Glashülle aufgedampft werden kann. Wie einleitend schon angedeutet hat jedoch das hellorange Licht eine sehr positive Wirkung, so dass das Filter für diese in einem Breich zwischen 580 und 620 nm liegende Strahlung vorteilhafterweise auch noch durchlässig sein sollte. Der Verlauf der Strahlungsintensität würde somit den in Figur 4 mit b bezeichneten Verlauf entsprechen.

Der Abstand zwischen Strahlungsquelle und Liege ist so zu wählen, dass sich der Anwender nicht beengt fühlt. In den meisten Fällen dürfte hierfür ein Abstand von 120 cm ausreichend sein.

Die erfindungsgemässe Art der IR-Bestrahlung lässt sich aber auch ohne weiteres mit UV-Bestrahlung kombinieren. Eine entsprechende UV-Strahlungsquelle kann entweder separat aufgebaut oder vorteilhafterweise gerade in die Infrarotlichtquelle integriert werden. Hierbei ist die Verwendung eines UV-B sowie eines UV-C-Filters empfehlenswert. Als UV-Strahlungsquelle kommt beispielsweise ein handelsüblicher Quecksilber-Niederdruckbrenner in Betracht.

## Ansprüche

1. Elektrisches Lichtbad zur flächenhaften Wärmebestrahlung eines menschlichen Körpers, mit mindestens einer Infrarotlichtquelle bestehend aus mindestens einem Strahlerelement und mindestens einem Reflektor, wobei durch das Strahlerelement vorwiegend eine Strahlung im Wellenbereich zwischen 760 und 1300 nm erfolgt, dadurch gekennzeichnet, dass für eine Bestrahlung von mehr als 50% und maximal 90% der Körperoberfläche, der Reflektor (R1) seitlich in Höhe eines Strahlerelementes (S) einen Spalt aufweist, und dass seitlich je ein Zusatzreflektor (R2) vorgesehen ist, welcher die durch den Spalt im erstgenannten Reflektor (R1) dringenden Strahlen auffängt und seitlich auf den Körper richtet, wobei die auf die Körperoberfläche einwirkende Bestrahlungsstärke mindestens 50 mW/cm², höchstens 150 mW/cm², vorzugsweise jedoch 60 bis 100 mW/cm² beträgt.

2. Lichtbad nach Anspruch 1, dadurch gekennzeichnet, dass der äussere Abstand der beiden Zusatzreflektoren (R2) wenigstens 60 cm, vorzugsweise jedoch 75 cm beträgt.

3. Lichtbad nach Anspruch 1 oder 2, dadurch gekennzeichnet, dass die Infrarotlichtquelle (Q) horizontal auf eine darunter angeordnete Liege (L) ausrichtbar ist.

4. Lichtbad nach einem der Ansprüche 1 bis 3, dadurch gekennzeichnet, dass der Abstand zwischen Infrarotlichtquelle (Q) und der Liege (L) veränderbar ist, vorzugsweise aber 120 cm beträgt.

5. Lichtbad nach einem der Ansprüche 1 bis 4, dadurch gekennzeichnet, dass wenigstens zwei seitlich von der Infrarotlichtquelle (Q) angeordnete, reflektierende Wände (R3) vorgesehen sind.

6. Lichtbad nach Anspruch 5, dadurch gekennzeichnet, dass wenigstens eine der reflektierenden Wände (R3) klappbar ist.

7. Lichtbad nach einem der Ansprüche 1 bis 6, dadurch gekennzeichnet, dass im unmittelbaren Bereich der Infrarotlichtquelle (Q) beabstandete, wenigstens annähernd parallel zum Strahlengang ausgerichtete Blendschutzlamellen (L) vorgesehen sind.

## Claims

1. An electric light bath for the areal irradiation of a human body with heat, with at least one infrared light source, consisting of at least one radiator element and at least one reflector, the radiator element producing radiation predominantly in the wave range between 760 and 1300 nm, characterised in that for an irradiation of more than 50% and at most 90% of the surface of the body, the reflector (R1) has a lateral slit at the height of a radiator element (S), and in that at each side an additional reflector (R2) is provided which intercepts the rays traversing the slit in the first-mentioned reflector (R1) and directs them onto the side of the body, the radiation intensity acting on the surface of the body being at least 50 mW/cm², at most 150 mW/cm², but preferably 60 to 100 mW/cm².

2. A light bath according to claim 1, characterised in that the outside distance of the two additional reflectors (R2) is at least 60 cm, but preferably 75 cm.

3. A light bath according to either of claims 1 or 2, characterised in that the infrared light source (Q) can be horizontally aligned relative to a bed (L) arranged underneath.

4. A light bath according to any of claims 1 to 3, characterised in that the distance between infrared light source (Q) and bed (L) is variable, but is preferably 120 cm.

5. A light bath according to any of claims 1 to 4, characterised in that at least two reflecting walls (R3) arranged to the sides of infrared light source (Q) are provided.

6. A light bath according to claim 5, characterised in that at least one of the reflecting walls (R3) can be swung.

7. A light bath according to any of claims 1 to 6, characterised in that spaced apart antiglare deflection plates (L), aligned at least approximately parallel to the path of rays, are provided in the immediate proximity of the infrared light source (Q).

## Revendications

1. Bain de lumière électrique pour l'irradiation thermique en surface d'un corps humain, comportant au moins une source de lumière infrarouge constituée d'au moins un élément générateur de rayonnement et d'au moins un réflecteur, cet élément générateur de rayonnement produisant principalement un rayonnement situé dans une gamme d'ondes comprise entre 760 et 1300 nm, caractérisé en ce que, pour une irradiation de plus de 50% et au maximum de 90% de la surface du corps, le réflecteur (R1) présente une fente, latéralement à hauteur d'un élément générateur de rayonnement (S), et que, de chaque côté, est prévu un réflecteur supplémentaire (R2), qui reçoit les rayons passant par la fente du réflecteur (R1) cité en premier et les dirige latéralement vers le corps, l'éclairement énergétique agissant sur la surface du corps s'élevant au minimum à 50 nW/cm², au maximum à 150 nW/cm², et de préférence à une valeur comprise entre 60 à 100 nW/cm².

2. Bain de lumière suivant la revendication 1, caractérisé en ce que l'écartement extérieur entre les deux réflecteurs supplémentaires (R2) est d'au moins 60 cm, mais de préférence de 75 cm.

3. Bain de lumière suivant la revendication 1 ou 2, caractérisé en ce que la source de lumière infrarouge (Q) disposée horizontalement est orientée vers un lit (L) disposé en dessous de celle-ci.

4. Bain de lumière suivant l'une quelconque des revendications 1 à 3, caractérisé en ce que la distance entre la source de lumière infrarouge (Q) et le lit (L) est variable, mais s'élève de préférence à 120 cm.

5. Bain de lumière suivant l'une quelconque des revendications 1 à 4, caractérisé en ce qu'au moins deux parois (R3) réfléchissantes disposées latéralement par rapport à la source de lumière infrarouge (Q) sont prévues.

6. Bain de lumière suivant la revendication 5, caractérisé en ce qu'au moins une des parois réfléchissantes (R3) est relevable.

7. Bain de lumière suivant l'une quelconque des revendications 1 à 6, caractérisé en ce que, dans la zone proche de la source de lumière infrarouge (Q), sont prévues des lames de protection contre l'éblouissement (L) espacées, orientées au moins en substance parallèlement à la trajectoire des rayons.

Fig. 1

Fig. 2

EP 0 275 817 B1

Fig. 3

Q

A

L

R₃

R₃

R'₃

Fig. 4